# EUROPÄISCHE PATENTSCHRIFT

(11) **EP 1 845 875 B1**
(45) Veröffentlichungstag und Bekanntmachung des Hinweises auf die Patenterteilung: **11.06.2008**
(21) Anmeldenummer: 06705936.0
(22) Anmeldetag: 08.02.2006
(51) Int. Cl.: A61B 17/70

(54) **WIRBELSÄULENFIXATEUR**
SPINAL FIXING DEVICE
SYSTEME DE CONTENTION DE LA COLONNE VERTEBRALE

(30) Priorität: 08.02.2005 DE 102005005647; 16.03.2005 US 661927 P
(43) Veröffentlichungstag der Anmeldung: 24.10.2007
(73) Patentinhaber: Kloss, Henning, 6373 Ennetbürgen (CH)
(72) Erfinder: SCHÄFER, Björn, 53809 Ruppichteroth (DE)
(74) Vertreter: Arth, Hans-Lothar
(86) Internationale Anmeldenummer: PCT/DE2006/000211
(87) Internationale Veröffentlichungsnummer: WO 2006/084443

(56) Entgegenhaltungen:
- WO-A-01/06940
- WO-A-03/096915
- DE-A1- 10 005 386
- DE-A1- 10 319 183
- US-A- 5 910 142
- US-A1- 2004 260 283

## Beschreibung

Die vorliegende Erfindung betrifft eine Vorrichtung zur Stabilisierung der Wirbelsäule umfassend mindestens zwei Pedikelschrauben und mindestens ein dazwischenliegendes Verbindungselement. Ferner betrifft die vorliegende Erfindung Pedikelschrauben, welche ein bewegliches Kugelelement in einem beweglichen Schraubenkopf für die leichtere Einführbarkeit des Verbindungselements aufweisen, wobei diese Konstruktion eine optimale Reponierbarkeit der Wirbel bei gleichzeitiger fester Einfassung des Verbindungselements ermöglicht.

Im Stand der Technik sind nur Ausführungsformen bekannt, welche eine auch nach der Implantation bewegliche Lagerung des Verbindungselementes im Schraubenkopf der Pedikelschraube entlang der axialen Achse beschreiben.

So offenbart US 2003/0220642 A1 in einer Ausführungsform ein elastisches Verbindungselement mit einem Gewinde und zugehörige Pedikelschrauben mit Schraubenköpfen mit entsprechendem Gewinde, so dass das Verbindungselement durch Drehbewegungen in die Schraubenköpfe eingeführt werden kann und entlang seiner Längsachse beweglich ist.

EP 0 669 109 B1 beschreibt eine Stützvorrichtung für die Wirbelsäule, welche weniger eine Bewegung des Verbindungselementes entlang seiner Längsachse zuläßt als vielmehr eine Bewegung der einzelnen Pedikelschrauben relativ zueinander gestattet. Zu diesem Zweck wird ein flexibles Band als Verbindungselement verwendet und ein wenig deformierbares Stützelement auf das Band aufgezogen, wobei das Stützelement fest an die Schraubenköpfe von jeweils zwei Pedikelschrauben anliegt.

US 6,761,719 B2 offenbart eine Stützvorrichtung für die Wirbelsäule, welche dadurch Bewegungen zuläßt, dass als Verbindungselement zwischen den Wirbeln ein Metall mit Formgedächtnis eingesetzt wird, welches bei Körpertemperatur pseudoelastische Eigenschaften aufweist.

Auch aus der WO 03/096915 sind Verankerungselemente bekannt, bei denen Pedikelschrauben Kugelsegmentförmige Kopfenden aufweisen, die in einer entsprechenden Halterung klemmbar gehalten sind.

Die in WO 03/037216 A2 genannten Ausführungsformen versuchen eine Beweglichkeit des Verbindungselementes zu erreichen, indem ein flexibles Verbindungselement aus beispielsweise Polyester, Polyethylen, Potylactid -oder Nitinol verwendet wird, welches jedoch in den Schraubenköpfen der Pedikelschrauben fixiert wird und dort keinerlei Bewegungsfreiheitsagrade besitzt.

Aufgabe der vorliegenden Erfindung ist die Bereitstellung einer Vorrichtung zur Stabilisierung der Wirbelsäule, welche das Verbindungselement fest einzufassen vermag und zum anderen eine optimale Reponierbarkeit der Wirbel ermöglicht.

Diese Aufgabe wird durch die Bereitstellung der zugehörigen Pediketschrauben gemäß Patentanspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die vorliegende Erfindung betrifft eine Pedikelschraube 1 umfassend einen Schraubenschaft 2 und einen Schraubenkopf 3 mit zwei gegenüberliegenden langlochförmigen Aussparungen, wobei in den Schraubenkopf 2 ein Kugelelement 6 mit einer Aussparung zur Aufnahme eines Verbindungselementes 4 beweglich gelagert werden kann. Dadurch wird erreicht, dass sich das Kugelelement 6 in Anteflexions-Retroflexions-Richtung der Lage des durchgeführten Verbindungselements 4 anpassen und dieses nach erfolgter Fixierung fest einschließen kann.

Die Ausgestaltung des Schraubenschafts 2 der Pedikelschraube 1 ist für die Erfindung nicht wesentlich und kann beliebige gängige Formen aufweisen. Die Pedikelschrauben 1 sind vorzugsweise selbstschneidend. In einer bevorzugten Ausführungsform weist der Schraubenschaft 2 ein Gewinde mit konstantem Außendurchmesser und mit einem konisch wachsenden Kerndurchmesser auf.

Wesentlich ist hingegen die Ausgestaltung des Schraubenkopfes 3 der Pedikelschraube 1 sowie des Kugelkopfes 17 des Schraubenschafts 2. Der Schraubenkopf 3 ist hohl und kann ein Kugelelement 6 aufnehmen, welches Drehbewegungen eines durchgeführten Verbindungselements 4 im Schraubenkopf 3 um die laterale Achse ermöglicht. Eine Drehbewegung des durchlaufenden Verbindungselements 4 um die laterale Achse bedeutet, dass das Verbindungselement 4 in Anteflexions- / Retroflexionsrichtung bewegt werden kann.

Damit nun das durchlaufende Verbindungselement 4 diese Bewegungen ausführen kann, muss der Schraubenkopf 3 zwei langlochförmige gegenüberliegende Öffnungen aufweisen, innerhalb derer die Kippbewegungen oder Neigebewegungen des Verbindungselements 4 ermöglicht werden. Daraus resultiert eine bevorzugte zylindrische bzw. ovale Ausgestaltung des Schraubenkopfes 3. Der Schraubenkopf 3 der Pedikelschraube 1 beansprucht nur ein geringes Volumen, wodurch ein tiefes Einschrauben ermöglicht wird und weist ferner keine scharfen Kanten auf.

Bezüglich der möglichen Bewegungen des Verbindungselements 4 im noch nicht fixierten Zustand werden die Achsen wie folgt bezeichnet. Als axiale Achse wird die entlang der Wirbelsäule verlaufende Achse bezeichnet. Die Anteflexions-Retroflexions-Achse verläuft senkrecht zur axialen Achse durch Bauch und Rücken des Patienten und die laterale Achse liegt senkrecht zur axialen Achse und ebenfalls senkrecht zur Anteflexions-Retroflexions-Achse.

Somit bewegt eine Drehung des Verbindungselements 4 um die durch den Schraubenkopf 3 verlaufende laterale Achse die Enden des Verbindungselements in Anteflexions-Retroflexions-Richtung und eine Drehung um die axiale Achse bewegt die durch das Verbindungselement 4 verlaufende Längsachse in laterale Richtung.

Das Kugelelement 6 ermöglicht somit eine Bewegung des Verbindungselement 4 im noch nicht fixierten Zustand in Anteflexions-Retroflexions-Richtung. Wichtig ist für eine vollständige Übertragung der Hebelkraft von Verbindungselement 4 auf die Pedikelschraube und weiter auf den Wirbelkörper, dass eine Beweglichkeit des Schraubenkopfes 3 auf dem Schraubenschaft 2 in Richtung der axialen Achse nicht gegeben ist. Derartige Bewegungen lassen Polyaxialschrauben zu, welche die Wirbel nur suboptimal reponieren können, wie weiter unten noch ausführlich ausgeführt wird.

Damit sich nun der Schraubenkopf 3 auf dem Schraubenschaft 2 nicht entlang der axialen Achse, sondern nur in Richtung der lateralen Achse bewegen kann, ist der Kopf 17 des Schraubenschafts 2 nicht als Kugel ausgebildet, um welche sich der Schraubenkopf 3 um 360 Grad drehen kann. Der Kopf 17 des Schraubenschafts 2 ist als Kugel mit zwei gegenüberliegenden, parallel zueinander verlaufenden Flächen (18a und 18b) ausgestaltet, wobei der Schraubenkopf 3 entsprechende parallele Flächen (18c ,18d) aufweist, welche an den Flächen 18a bzw. 18b anliegen. Die Flächen 18a und 18b sind gleich groß. Die Flächen 18c und 18d sind ebenfalls gleich groß und alle Flächen 18a, 18b, 18c und 18d sind -parallel zueinander. Dadurch wird eine Kippbewegung des Schraubenkopfes 3 auf dem Schraubenschaft 2 ermöglicht, jedoch nur in lateraler Richtung, also um die axiale Achse. Somit kann der auf dem Schraubenschaft 2 erfindungsgemäß gelagerte Schraubenkopf 3 nur Neigebewegungen in Richtung der lateralen Achse, d.h. in der durch die laterale Achse und die Anteflexions-Retroflexions-Achse aufgespannte zur axialen Achse senkrecht verlaufende Ebene ausführen. Hingegen ist der auf dem Schraubenschaft 2 gelagerte Schraubenkopf 3 nicht frei um die Längsachse des Schraubenschafts 2 drehbar.

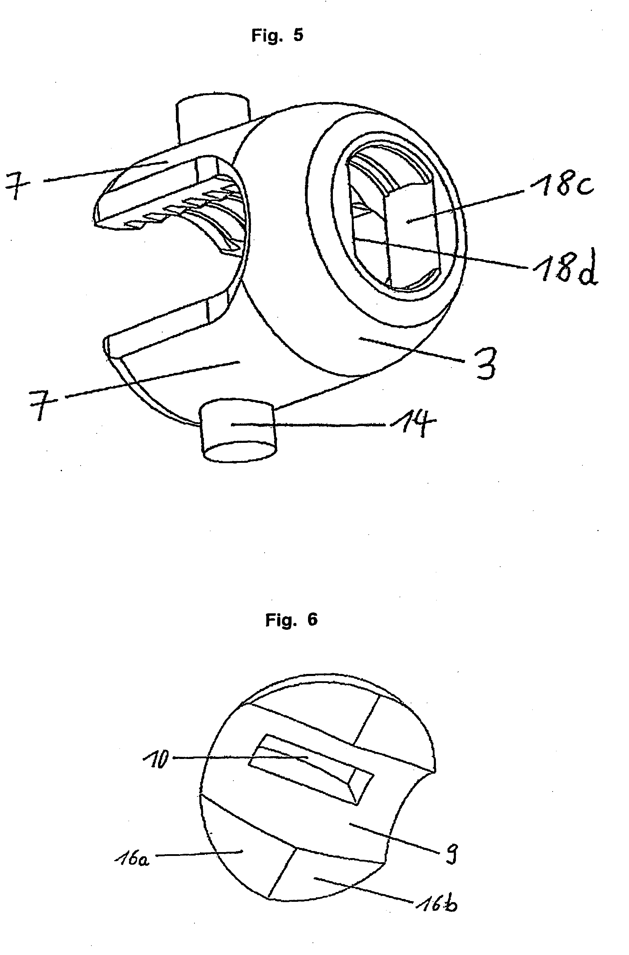

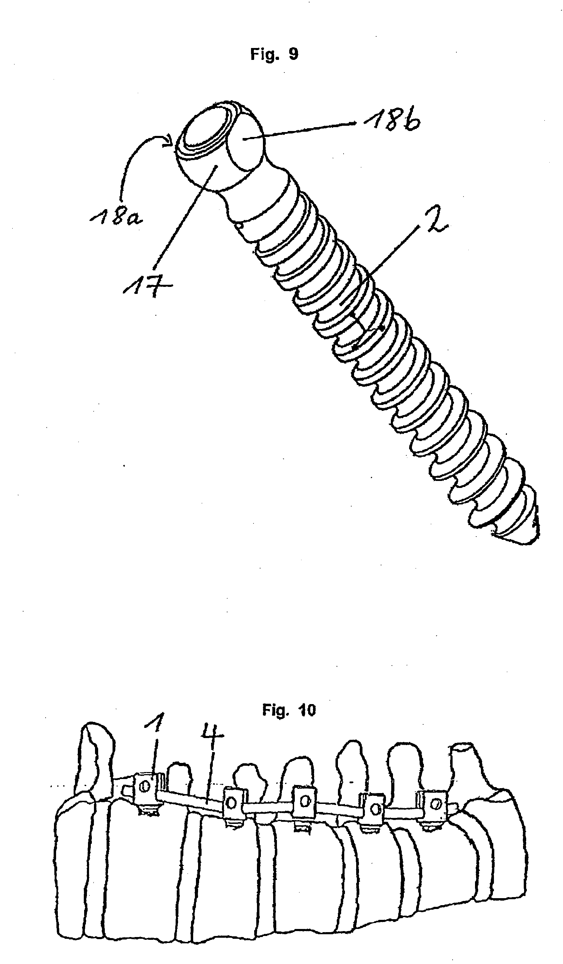

Die erfindungsgemäße Ausgestaltung des Kugelkopfes 17 in Form einer Kugel mit zwei gegenüberliegenden parallelen Flächen bezeichnet als 18a und 18b ist in Figur 9 dargestellt. Diese an den entsprechenden parallelen Flächen 18c und 18d des Schraubenkopfes 3 anliegenden Flächen 18a und 18b verhindern eine Rotationsbewegung um die axiale Achse und erlauben nur eine Neigebewegung von bis zu 45° bevorzugt von bis zu 30° ausgehend von der zentralen Mittelstellung in jeweils eine laterale Richtung. Die parallelen flächen 18c und 18d sind in Figur 5 gut zu erkennen.

Die parallelen Flächen 18c und 18d befinden sich am unteren dem Schraubenschaft 2 zugewandten Ende des Schraubenkopfes 3, wie in Figur 5 gezeigt. Das Gewinde im Schraubenkopf 3 erstreckt sich nicht in diesen Bereich. Der Durchmesser des Schaftes des Schraubenschafts 2 ist geringer als der Abstand der parallelen Flächen 18c und 18d zueinander, so dass der Schraubenschaft 2 in die untere Öffnung des Schraubenkopfes 3 so weit eingeführt werden kann, bis die parallelen Flächen 18a, 18c sowie 18b und 18d aneinander liegen und der Kugelkopf 17 den Boden zur Aufnahme des Kugelelements 6 bildet.

Diese limitierte Bewegung ist zum einen sehr wichtig, um die Wirbel optimal zu repositionieren und zum anderen, um das Verbindungselement 4 bei der Fixierung durch das Fixierungsmittel 5 fest einzufassen. Die erfindungsgemäßen Pedikelschrauben vereinen nämlich in geschickter Form die positiven Eigenschaften einer Monoaxialschraube mit denen einer Polyaxialschraube unter gleichzeitiger Vermeidung der unerwünschten Eigenschaften beider Schraubentypen. Die erfindungsgemäße Pedikelschraube übernimmt bei der Implantation die Funktion einer Monoaxialschraube, d.h. gewährleistet eine optimale Reponierbarkeit und weist zum Zeitpunkt der Einführung des Verbindungselements die Vorteile einer Polyaxialschraube auf, wodurch die Einführung und Festlegung des Verbindungselements deutlich erleichtert wird, ohne diesen Vorteil durch schlechtere Reponierbarkeit.erkaufen zu müssen.

Traumatisierte Wirbelsäulen werden über einen Fixateur in ihre Ursprungsform zurückgebracht und dort gehalten. Dies geschieht, indem der Operateur die Pedikelschraube in den Pedikel des Wirbels einbringt und über die Schraube als Hebel, den Wirbel in die optimale Position reponiert. Dieses Zurückbringen der Wirbel in ihre jeweilige optimale Position (Reponierbarkeit) vermögen Monoaxialschrauben am besten zu bewerkstelligen. Aufgrund des starren Schaubenkopfes bei Monoaxialschrauben kann das Verbindungselement 4 teilweise nur schwer vollständig und in unbeweglicher Weise eingefaßt werden, so dass bei der Implantation höchste Präzision von Nöten ist. Ist jedoch der Schraubenkopf polyaxial gelagert, kann sich der Schraubenkopf der Lage des Verbindungselements 4 anpassen und dieses fest einschließen. Leider bringt diese Beweglichkeit des Schraubenkopfes den Nachteil mit sich, dass die Wirbel nicht optimal reponiert werden können. Polyaxiale Schrauben sind in der Regel über eine kugelförmige Aufnahme des Schraubenkopfes in alle Richtungen beweglich gelagert mit dem Gewindestab, d.h. dem Schfaubenschaft der Pedikelschraube verbunden.

Ist also der Schraubenkopf polyaxial gelagert, so besteht nur eingeschränkt die Möglichkeit, die Pedikelschraube als Hebel einzusetzen, um den Wirbel in die optimale Position zu reponieren, da die Hebelwirkung nicht oder nur ungenügend auf die Schaftachse der Pedikelschraube übertragen wird. Monoaxiale Pedikelschrauben bieten zwar diese Möglichkeit weisen jedoch andere Nachteile auf, wie beispielsweise sehr exaktes und zeitaufwendiges Arbeiten beim Anformen des Stabilisierungsstabes (Verbindungselement 4). Bei entsprechender Indikation besteht eine Verteilung von 50% : 50% bei der Wahl der Chirurgen von mono- bzw. polyaxialen Systemen.

Die Monoaxialschraube vermag ein Verbindungselement 4 nach erfolgter Fixierung teilweise nicht fest einzufassen, erfordert zudem eine sehr hohe Präzision bei der Implantation, bietet jedoch den Vorteil einer optimalen Reponierbarkeit der Wirbel. Hingegen bietet die Polyaxialschraube eine feste Einfassung des Verbindungselements 4 bei weniger präzise erfolgter Implantation der einzelnen Pedikelschrauben mit dem Nachteil, dass aufgrund der auch nach der Implantation weiterhin vorhandenen Beweglichkeit des Schraubenkopfes die Wirbel nur suboptimal reponiert werden können.

Die erfindungsgemäßen Ausführungsformen erreichen durch das Kugelelement 6, d.h. das kugelförmige Aufnahmemittel für das Verbindungselement 4 ein festes Einschließen bzw. Einfassen des Verbindungselements 4 (Stabilisierungsstab)bei der Fixierung, wobei während der Implantation die gewünschte .Beweglichkeit bereitgestellt wird, so dass die Implantation der Pedikelschrauben einfacher erfolgen kann, da keine extreme Präzision erforderlich ist und dennoch die Reponierbarkeit (die Reposition) einer Monoaxialschraube gegeben ist, da aufgrund der Unbeweglichkeit des Schraubenkopfes insbesondere entlang der kritischen axialen Achse die volle Hebelwirkung auf die Schaftachse der Pedikelschraube übertragen werden kann.

Bei sehr bevorzugten Ausführungsformen der vorliegenden Erfindung ist auch der Schraubenkopf 3 auf dem Schraubenschaft 2 beweglich gelagert, jedoch nicht wie bei Polyaxialschrauben in jede Richtung bewegbar bzw. um 360° drehbar. Erfindungsgemäß sitzt der Schraubenkopf 3 auf dem Schraubenschaft 2, der einen Kugelkopf 17 mit abgeflachten Seiten 18a und 18b aufweist, so dass der Schraubenkopf 3 nur um die axiale Achse beweglich ist (s. Fig. 5 + 9). Diese Beweglichkeit kombiniert mit der Beweglichkeit durch das Kugelelement 6 verleiht der Pedikelschraube und dadurch der gesamten Vorrichtung die für eine feste, unbewegliche Lagerung des Verbindungselements 4 erforderliche Beweglichkeit im nicht fixierten Zustand zusammen mit einer für die optimale Reponierbarkeit der Wirbel erforderlichen Starrheit.

Am Schraubenkopf 3 der Pedikelschraube 1 können Haltemittel 14 in Form von Stiften, Auswölbungen, Einkerbungen oder Aussparungen angebracht sein, um ein Eindrehwerkzeug oder ein Halteinstrument ansetzen zu können.

Zur leichteren Einführung des Kugelelements 6 in den Schraubenkopf 3 ist es zudem bevorzugt, dass der Schraubenkopf 3 keine Durchgangsbohrung entlang der axialen Achse, sondern eine nach oben offene Aussparung aufweist. Ferner ist es für eine einfache Lagerung und Fixierung des Kugelelements 6 vorteilhaft, wenn der Schraubenkopf 3 entlang der Achse des Schraubenschafts 2 eine Bohrung aufweist, deren Innendurchmesser weitgehend dem Außendurchmesser des Kugelelements 6 entspricht. Durch die Bohrung entsteht eine zentrierte Öffnung im Schraubenkopf 3 an der dem Schraubenschaft 2 gegenüberliegenden Seite, welche zur Aufnahme eines Fixierungsmittels 5 geeignet ist.

Figur 5 zeigt den Schraubenkopf 3 der Pedikelschraube 1 mit der inneren Bohrung und der U-förmigen Aussparung, welche nach dem Einsetzen der Pedikelschraube 1 in einen Wirbelkörper entlang der Wirbelsäulenachse, d.h. der axialen Achse verlaufen sollte. Der Schraubenkopf 3 weist in den bevorzugten Ausführungsformen zwei halbkreisförmige gegenüberliegende Schenkel 7 auf, zwischen denen das Kugelelement 6 mit durchgeführtem Verbindungselement 4 gelagert werden können. In einer bevorzugten Ausführungsform weist der Schraubenkopf 3 am Boden eine Aussparung auf, welche zwei halbkreisförmig gegenüberliegende Seiten und zwei parallel zueinander verlaufende Seiten besitzt. In diese Aussparung wird ein Kugelkopf mit zwei entsprechend -parallel zueinander liegenden Flächen eingeführt, so dass nur eine Neigebewegung aber keine Rotationsbewegung des Schraubenkopfes 3 auf dem Schraubenschaft 2 möglich wird.

Als Kugelelement 6 kommen beliebige um die laterale Achse bewegliche Elemente in Frage. Vorteilhaft sind Kugeln mit einer zentrierten Durchgangsbohrung zur Aufnahme des Verbindungselements 4. insbesondere bevorzugt sind zweiteilige Kugelelemente 6, welche beispielsweise aus einer unteren, dem Schraubenschaft 2 zugewandten Olive 8 und einer oberen, dem Fixierungsmittel zugewandten Olive 9 bestehen. Die Oliven 8 und 9 weisen einen Innendurchmesser auf, der bevorzugt gleich dem Außendurchmesser des Verbindungselements 4 ist. Ferner weisen die Oliven 8 und 9 vorzugsweise einen Außendurchmesser auf, der weitgehend gleich dem Innendurchmesser der Bohrung im Schraubenkopf 3 in Richtung der Längsachse des Schraubenschaftes 2 ist.

In einer weiteren bevorzugten Ausführungsform sind die Auflageflächen 15 und 16 beider Oliver 8 und 9 nicht planparallel sondern gewinkelt ausgestaltet.

Fig. 6 zeigt eine mögliche Ausgestaltungsform der oberen Olive 9 mit angeschrägten Auflageflächen 16 für die untere Olive 8. Die beiden Teilflächen 16a und 16b der Auflagefläche 16 schließen einen Winkel von 100° bis 190°, bevorzugt von 110 bis 180 Grad und insbesondere bevorzugt von 115 bis 175 Grand ein. Die untere Olive 8 besitzt die gleiche Form wir die obere Olive 9, weist jedoch keinen Aussparung 10 auf. Auch die beiden Teilflächen 15a und 15b der Auflagefläche 15 der unteren Olive 8 können einen Winkel von 100° bis 190°, bevorzugt von 110 bis 180 Grad und insbesondere bevorzugt von 115 bis 175 Grand miteinander einschließen. In einer bevorzugten Ausführungsform liegt die untere Olive 8 auf dem Kugelkopf 17 des Schraubenschaftes 2 auf.

Die Innenflächen des Kugelelements 6 bzw. die Innenflächen der unteren Olive 8 als auch der oberen Olive 9, welche an das Verbindungselement 4 anliegen, können rau ausgestaltet sein oder eine unebene Oberflächenstruktur aufweisen, um das Verbindungselement 4 fest einzuschließen und Translationsbewegungen des Verbindungselements 4 innerhalb des Kugelelements 6 nach der Implantation zu verhindern.

Bei den bevorzugten Ausführungsformen der Pedikelschraube 1 mit einer Bohrung im Schraubenkopf 3 in Richtung der Längsachse des Schraubenschafts 2 ist es notwendig, das Kugelelement 6 mit einem Fixierungsmittel 5 zu sichern: Als Fixierungsmittel können Stifte, Bolzen, Stangen, Keile oder andere Mittel eingesetzt werden, welche das Kugelelement 6 im Schraubenkopf 3 fixieren. Als insbesondere vorteilhaft haben sich Gewindeschrauben oder Gewindestifte als Fixierungsmittel 5 erwiesen. Somit ist es ferner bevorzugt, die Bohrung im Schraubenkopf 3 zumindest im oberen Teil als Gewindebohrung auszugestalten. Durch das Fixierungsmittel 5 wird das Verbindungselement 4 in dem Kugelelement 6 fest und unbeweglich eingeschlossen bzw. eingefasst. Nach erfolgter Fixierung, insbesondere nach erfolgtem Anziehen der Gewindeschrauben wird die erfindungsgemäße Vorrichtung in ihrer Lage fixiert, so dass die bisher beweglichen Elemente der einzelnen Pedikelschrauben in ihrer jeweiligen Lage starr und unbeweglich verharren. Eine Beweglichkeit der Vorrichtung beruht in diesem Zustand nur noch auf der Flexibilität des Verbindungselements 4.

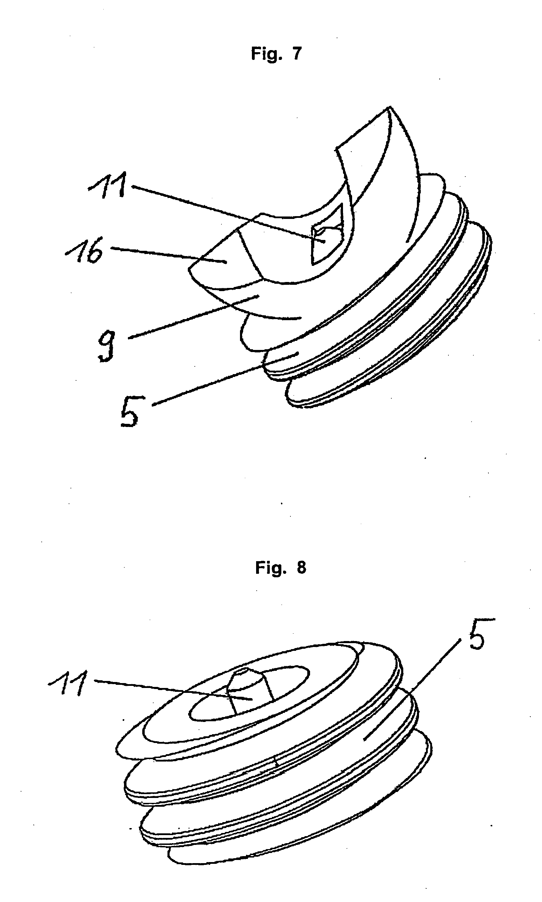

Fig. 8 zeigt ein bevorzugtes erfindungsgemäßes Fixierungsmittel 5 in Form einer Gewindeschraube oder Gewindestiftes, welche in ihrem Kopf eine Aussparung 12 für den Einsatz eines Schraubwerkzeugs vorsieht. Im vorliegenden Fall ist eine Sechskantaussparung für die Verwendung eines Sechskantschraubenschlüssels vorgesehen. Ferner weist das Fixierungsmittel 5 vorzugsweise an seiner Spitze einen Führungsstift 11 auf, der innerhalb einer entsprechenden Aussparung in dem Kugelelement 6, vorzugsweise in der oberen Olive 9 gleitend beweglich ist.

Fig. 7 zeigt, wie die obere Olive 9 auf dem Fixierungsmittel 5 aufliegt und der Führungsstift 11 am Fixierungsmittel 5 in die entsprechende Aussparung in der oberen Olive 9 eingreift.

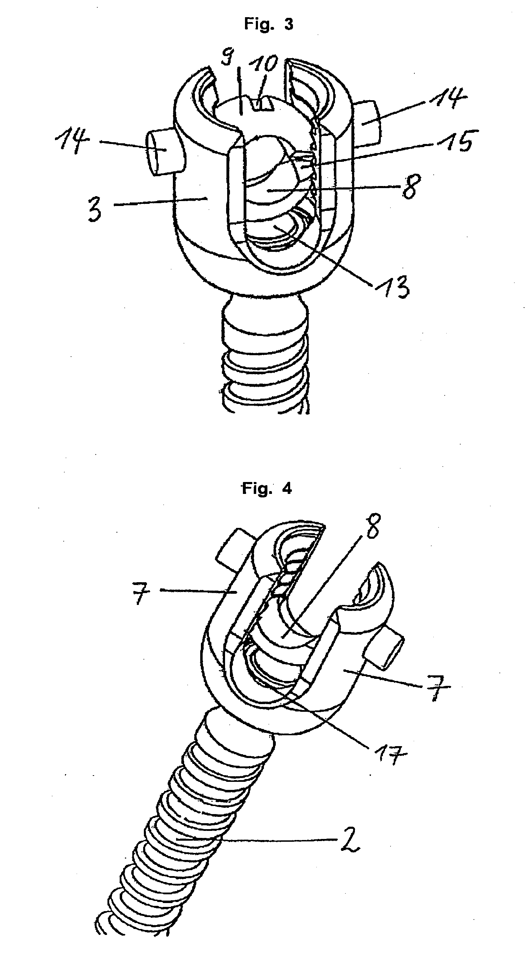

Fig. 4 zeigt den Schraubenkopf 3 mit eingesetzter unterer Olive 8. Damit die untere Olive 8 die Schwenkbewegungen um die laterale Achse einfacher ausführen kann, ist es bevorzugt, die untere Olive 8 bzw. das Kugefelement 6 auf dem Boden des Schraubenkopfes 3 auf einer mittigen konvexen Wölbung 13 zu lagern. Somit weist der Schraubenkopf 3 an seiner dem Schraubenschaft 2 zugewandten Seite vorzugsweise eine zentrierte konvexe Wölbung 13 auf, welche insbesondere als Halbkugel ausgebildet ist und als punktförmige Auflagefläche für das Kugelelement 6 bzw. die untere Olive 8 dient. Bei den insbesondere bevorzugten Ausführungsformen mit in Richtung der lateralen Achse beweglichem Schraubenkopf 3 handelt es sich bei dieser konvexen Wölbung 13 um den Kugelkopf 17.

Einen erfindungsgemäßen Schraubenkopf 3 mit eingesetztem Kugelelement 6 in Form einer unteren Olive 8 und oberen Olive 9 ist in Fig. 3 abgebildet. Die zylinderförmige Durchgangsbohrung im Kugelelement 6 erstreckt sich durch die beiden Aussparungen in Form von Langlöchern im Schraubenkopf 3.

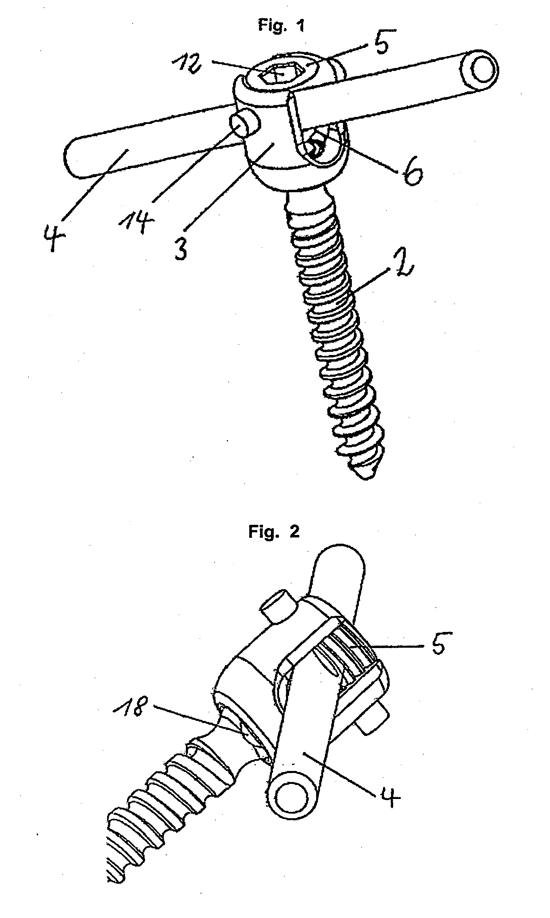

Führt man nun das Verbindungselement 4 in die Durchgangsbohrung im Kugelelement 6 ein, so kann sich das Verbindungselement 4 im Langloch des Schraubenkopfes 3 um die laterale Achse bewegen, solange das Verbindungselement 4 und gleichzeitig das Kugelelement 6 nicht durch das Fixierungsmittel 5 in der jeweiligen Position fixiert worden ist. Wird nundas in die Durchgangsbohrung im Kugelelement 6 eingeführte Verbindungselement 4 mittels des Fixierungsmittels 5 im Schraubenkopf 3 festgelegt, eingefaßt oder eingeklemmt, so zeigt Fig. 2 deutlich, dass das Verbindungselement 4 sich im Langloch des Schraubenkopfes 3 nicht mehr bewegen kann und fest eingeschlossen bzw. eingeklemmt ist. Eine Drehbewegung bzw. Rotationsbewegung um die laterale Achse kann im festgelegten Zustand nicht mehr erfolgen. Gleiches gilt für den entlang der lateralen Achse auf dem Schraubenschaft 2 beweglich gelagerten Schraubenkopf 3. Ferner sind auch jegliche Translationsbewegungen entlang der Wirbelsäulenachse, d.h. entlang der axialen Achse im festgelegten, eingefaßten oder eingeklemmten Zustand nicht mehr möglich.

Als Materialien für die erfindungsgemäßen Pedikelschrauben 1 können verwendet werden: medizinischer Edelstahl, Titan oder Titanlegierungen, Tantal, Chrom, Cobalt-Chrom-Legierungen, Vanadium, Wolfram, Molybdän, Kunststoffe wie beispielsweise PEEK (Polyetheretherketon) sowie faserverstärkte Kunststoffe.

Als Verbindungselemente 4 können Stützstäbe, Rohre, Drahtgeflechte, Führungsschienen oder Führungsstäbe eingesetzt werden. Es ist ferner bevorzugt, wenn das mindestens eine Verbindungselement 4 gebogen ist oder biegsam ist, um dem Verlauf der Wirbelsäule angepaßt werden zu können.

Als Materialien für das mindestens eine Verbindungselement 4 kommen in Betracht: medizinischer Edelstahl; Titan oder Titanlegierungen, Tantal, Chrom, Cobalt-Chrom-Legierungen, Vanadium, Wolfram, Molybdän, Kunststoffe wie beispielsweise PEEK (Polyetheretherketon) sowie faserverstärkte Kunststoffe.

Ferner ist bevorzugt, wenn die einzelnen Komponenten der erfindungsgemäßen Pedikelschraube 1 oder zumindest deren Kontaktflächen mit einer keramischen Beschichtung überzogen sind. Keramische Beschichtungen umfassen Nitride, Carbide und Phosphide von bevorzugt Halbmetallen und Metallen bzw. Metalllegierungen. Beispiele für keramische Beschichtungen sind Bornitride, Titan-Niob-Nitrid, Titan-Calcium-Phosphid (Ti-Ca-P), Cr-AI-N, Ti-Al-N, Cr-N, TiAIN-CrN, Ti-Al-C, Cr-C, TiAIC-CrC, Zr-Hf-N, Ti-Hf-C-N, Si-C-N-Ti, Si-C-N sowie DLC (Diamond Like Carbon). Als Beschichtung wird ferner vorzugsweise eine keramische Schicht aus Titan-Niob-Nitrid (Ti-Nb-N) aufgebracht.

Insbesondere ist es vorteilhaft, wenn die Kontaktflächen -der Einzelkomponenten mit Titan-Niob-Nitrid (Ti-Nb-N) beschichtet sind. Eine keramische Beschichtung aus Titan-Niob-Nitrid hat eine Härte, die um ein Vielfaches höher ist, als die von herkömmlich verwendeten Materialien. Durch diese Härte ist die Oberfläche hoch polierbar und vor Titanabtrieb geschützt.

Des weiteren betrifft die vorliegende Erfindung eine Vorrichtung zur Stabilisierung der Wirbelsäule, welche aus einer Vielzahl aber zumindest zwei erfindungsgemäßen Pedikelschrauben 1 und mindestens einem Verbindungselement 4 besteht.

Die erfindungsgemäße Vorrichtung zur Stabilisierung der Wirbelsäule ist nicht nur geeignet, um zwei benachbarte Wirbelkörper mit dazwischenliegender Bandscheibe zu stabilisieren, sondern kann über mehrere Wirbelkörper hinweg angebracht werden, um größere Bereiche der Wirbelsäule zu stützen.

In Kontakt stehen in der Regel mindestens 2 Pedikelschrauben, verbunden durch das Verbindungselement 4, d.h. einen Stabilisierungsstab. Hierdurch wird die Wirbelsäule durch externe Elemente stabilisiert (ggf. korrigiert). Traumatisierte oder degenerativ veränderte Wirbel werden reponiert und in korrigierter Position durch den Wirbelsäulenfixateur in ihrer räumlichen Anordnung gehalten. Außer der Eigenflexibilität des Verbindungselements 4 (Stabilisierungsstabes) wird der traumatisierte (bzw. degenerierte) Wirbelsäulenbereich weitestgehend fixiert, sodass die Wirbelsäule an sich, ihre tragende Funktion ausüben kann, auch wenn geschädigte Strukturen vorliegen. Die geschädigten Strukturen werden dadurch entlastet und eine erneute Fehlpositionierung wird verhindert.

### Abkürzungsverzeichnis:

- 1: Pedikelschraube
- 2: Schraubenschaft
- 3: Schraubenkopf
- 4: Verbindungselement
- 5: fixierungsmittel
- 6: Kugelelement
- 7: Schenkel
- 8: untere -Olive
- 9: obere Olive
- 10: Aussparung in oberer Olive
- 11: Führungsstift am fixierungsmittel
- 12: Aussparung für Ansatz von Werkzeug
- 13: konvexe Wölbung am Boden des Schraubenkopfes
- 14: Haltemittel für Ansatz von Schraubwerkzeug
- 15: Auflagefläche der unteren Olive 8
- 15a: Teilfläche der Auflagefläche der unteren Olive 8
- 15b: Teilfläche der Auflagefläche der unteren Olive 8
- 16: Auflagefläche der oberen Olive 9
- 16a: Teilfläche der Auflagefläche der oberen Olive 9
- 16b: Teilfläche der Auflagefläche der oberen Olive 9
- 17: Kugelkopf des Schraubenschafts 2
- 18: parallelen Flächen des Kugelkopfes 17
- 18a: parallele Fläche A des Kugelkopfes 17
- 18b: parallele Fläche B des Kugelkopfes 17
- 18c: parallele Fläche A des Schraubenkopfes 3
- 18d: parallele Fläche B des Schraubenkopfes 3

### Figurenbeschreibung

- Figur 1: zeigt eine Seitenansicht einer Ausführungsform der erfindungsgemäßen Pedikelschraube 1 mit eingesetztem Verbindungselement 4;
- Figur 2: zeigt eine Ansicht der Pedikelschraube 1 entlang der Längsachse des Verbindungselements 4;
- Figur 3: zeigt den Schraubenkopf 3 der Pedikelschraube 1 mit eingesetztem Kugelelement 6;
- Figur 4: zeigt den Schraubenkopf 3 der Pedikelschraube 1 mit eingesetzter unterer Olive 8 als Teil des Kugelelements 6 sowie eine der beiden abgeflachten Seiten des Kopfes 17 des Schraubenschafts 2;
- Figur 5: zeigt den hohlen Schraubenkopf 3 der Pedikelschraube 1 in U-förmiger Ausgestaltung mit den beiden Schenkeln 7; am Boden des Schraubenkopfes 3 ist die längliche Aussparung mit zwei zueinander parallelen Seiten (18c, 18d) zur Aufnahme des entsprechend ausgestalteten Kugelkopfes 17 des Schraubenschafts zu erkennen;
- Figur 6: zeigt eine mögliche Ausgestaltung einer oberen Olive -9 als Teil eines Kugelelements 6; die untere Olive weist eine ähnliche Ausgestaltung wie die obere Olive jedoch ohne Aussparung 10 auf;
- Figur 7: zeigt eine Ausgestaltungsform der oberen Olive 9 als Teil des Kugelelements 6, welche auf dem Fixierungsmittel 5 aufliegt;
- Figur 8: zeigt ein Fixierungsmittel 5 in Form eines Gewindestiftes;
- Figur 9: zeigt den Schraubenschaft 2 mit dem Kugelkopf 17 und den beiden flachen parallel zueinander liegenden Flächen 18a und 18b;
- Figur 10: zeigt eine Ausführungsform der erfindungsgemäßen Vorrichtung zur Stabilisierung der Wirbelsäule.

### Ausführungsbeispiele

Bevorzugte Ausführungsformen der erfindungsgemäßen Pedikelschraube bzw. Vorrichtung werden nun anhand der Beispiele diskutiert, wobei zu berücksichtigen ist, dass die diskutierten Beispiele vorteilhafte Ausgestaltungen der Erfindung wiedergeben, jedoch den Schutzumfang nicht auf diese Ausführungsformen beschränkten.

### Beispiel 1

Eine Pedikelschraube 1 einer Gesamtlänge von 25 - 60 mm wird bereitgestellt. Die Pedikelschraube 1 besteht aus Titan. Ihr Schraubenschaft 2 besitzt eine der Größe der Pedikelschraube angepasste Länge von 15 - 45 mm und einen Außendurchmesser von 13 - 15 mm. Der Schraubenkopf 3 weist einen Außendurchmesser von 17 - 20 mm auf.

Der Schraubenkopf 3 ist oval ausgestaltet und weist eine 10 - 13 mm tiefe zentrierte Bohrung in Richtung der Längsachse des Schraubenschafts 2 auf, welche im oberen Bereich des Schraubenkopfes 3 als Gewindebohrung ausgestaltet ist. Der Innerdurchmesser dieser Bohrung beträgt 6 - 8 mm.

Der Kugelkopf 17 des Schraubenschafts 2 ist nicht als vollständige Kugel ausgestaltet, sondern als Kugel mit zwei parallelen gegenüberliegenden Flächen (18a, 18b), welche mit den entsprechenden Flächen 18c und 18d des Schraubenkopfes 3 korrespondieren und dessen Beweglichkeit bestimmen bzw. die freie Drehbarkeit verhindern. Die parallelen gegenüberliegenden flächen 18a als auch 18b besitzen jeweils eine Fläche von ca. 0,7 cm².

Der Schraubenkopf 3 weist ferner eine nach oben offene langlochförmige und am Boden des Schraubenkopfes 3 rund ausgestaltete Durchgangsbohrung auf, welche einen Durchmesser hat, der dem Durchmesser des einzuführenden Verbindungselements 4 weitgehend entspricht.

Am Boden des Schraubenkopfes 3 befindet sich eine halbkugelförmige konvexe Wölbung 13, auf welcher die untere Olive 8 aufliegt. Innendurchmesser der unteren Olive 8 als auch der oberen Olive 9 entsprechen weitgehend dem Außendurchmesser des einzuführenden Verbindungselements 4.

Auf die untere Olive 8 wird die Obere Olive 9 aufgesetzt, wobei die Auflageflächen 15a, 15b bzw. 16a, 16b beider Oliver einen Winkel von 150° zueinander einschließen.

Die obere Olive 9 weist eine längliche Aussparung 10 entlang der axialen Achse auf, welche den Führungsstift 11 des Fixierungsmittels 5 aufnimmt. Der Führungsstift 11 hat eine Länge von 1 - 3 mm und einen Außendurchmesser von 0,5 - 2 mm.

Auf die obere Olive 9 wird das Fixierungsmittel 5 in Form eines Gewindestiftes aufgesetzt. Der Gewindestift weist an seinem unteren Ende den Führungsstift 11 und in seinem Kopf eine Aussparung zur Ausnahme eines Eindrehwerkzeugs auf.

Der Gewindestift besitzt ein der Gewindebohrung im Schraubenkopf 3 entsprechendes Gewinde und kann in den Schraubenkopf 3 hineingedreht werden, bis er das Verbindungselement 4 fest zwischen die obere Olive 9 und untere Olive 8 einschließt.

Als Verbindungselement 4 dient ein biegbares Rohr aus medizinischem Edelstahl, Titan, Titanlegierungen oder Tantal, mit einer variablen Länge von 4 cm bis zu 30 cm und einem Außendurchmesser von 3-8 mm.

Das Verbindungselement 4 kann sich nicht entlang der Wirbelsäulenachse translatorisch bewegen, vermag aber solange es nicht fixiert ist eine Drehbewegung von bis zu 12 Grad, bevorzugt von bis zu 24 Grad um die laterale Achse auszuführen, ausgehend von einer horizontalen Lage, welche senkrecht zur Längsachse des Schraubenschafts verläuft.

Die Einzelkomponenten der Pedikelschraube 1 sind vorzugsweise mit einer keramischen Beschichtung versehen.

### Beispiel 2

Eine Pedikelschraube 1 einer Gesamtlänge von 45 mm wird bereitgestellt. Die Pedikelschraube 1 besteht aus Tantal. Ihr Schraubenschaft 2 besitzt eine der Größe der Pedikelschraube angepasste Länge von 35 mm und einen Außendurchmesser von ca. 14 mm. Der Schraubenkopf 3 weist einen Außendurchmesser von ca. 18 mm auf.

Der Schraubenkopf 3 ist oval ausgestaltet und weist eine ca. 12 mm tiefe zentrierte Bohrung in Richtung der Längsachse des Schraubenschafts 2 auf, welche im oberen Bereich des Schraubenkopfes 3 als Gewindebohrung ausgestaltet ist. Der Innerdurchmesser dieser Bohrung beträgt ca. 7 mm.

Der Kugelkopf 17 des Schraubenschafts 2 ist nicht als vollständige Kugel ausgestaltet, sondern als Kugel mit zwei parallelen gegenüberliegenden Flächen (18a, 18b), welche mit den entsprechenden Flächen 18c und 18d des Schraubenkopfes 3 korrespondieren und dessen Beweglichkeit bestimmen bzw. die freie Drehbarkeit verhindern. Die parallelen gegenüberliegenden Flächen 18a als auch 18b besitzen jeweils eine Fläche von ca. 0,55 cm².

Der Schraubenkopf 3 weist ferner eine nach oben offene langlochförmige und am Boden des Schraubenkopfes 3 rund ausgestaltete Durchgangsbohrung auf, welche einen Durchmesser hat, der dem Durchmesser -des einzuführenden Verbindungselements 4 weitgehend entspricht.

Der Schraubenschaft 2 ist mit einem Kugelkopf 17 versehen, wobei der Kugelkopf 17 zwei gegenüberliegende, parallele Flächen 18a und 18b aufweist. Der Schraubenkopf 3 ist mit einer entsprechenden Aussparung zur Aufnahme des Kugelkopfes 17 versehen und auf dem Schraubenschaft 2 in lateraler Richtung beweglich gelagert. Der Schraubenkopf 3 kann ausgehend von der zentrierten Position in laterale Richtung um bis zu 30 Grad geneigt werden. Den Boden des Schraubenkopfes 3 bildet somit der Kugelkopf 17, auf dem die untere Olive 8 aufliegt. Innendurchmesser der unteren Olive 8 als auch der oberen Olive 9 entsprechen weitgehend dem Außendurchmesser des einzuführenden Verbindungselements 4.

Auf die untere Olive 8 wird die Obere Olive 9 aufgesetzt, wobei die Auflageflächen 15a, 15b bzw. 16a, 16b beider Oliver einen Winkel von 140° zueinander einschließen.

Die obere Olive 9 weist eine längliche Aussparung 10 entlang der axialen Achse auf, welche den Führungsstift 11 des Fixierungsmittels 5 aufnimmt. Der Führungsstift 11 hat eine Länge von 1-3 mm und einen Außendurchmesser von 0,5-2 mm.

Auf die obere Olive 9 wird das Fixierungsmittel 5 in Form eines Gewindestiftes aufgesetzt. Der Gewindestift weist an seinem unteren Ende den Führungsstift 11 und in seinem Kopf eine Aussparung zur Ausnahme eines Eindrehwerkzeugs auf.

Der Gewindestift besitzt ein der Gewindebohrung im Schraubenkopf 3 entsprechendes Gewinde und kann in den Schraubenkopf 3 hineingedreht werden, bis er das Verbindungselement 4 fest zwischen die obere Olive 9 und untere Olive 8 einschließt.

Als Verbindungselement 4 dient ein biegbares Rohr aus medizinischem Edelstahl mit einer Länge von 25 cm und einem Außendurchmesser von 6 mm.

Das Verbindungselement 4 kann sich nicht entlang der Wirbelsäulenachse translatorisch bewegen, vermag aber solange es nicht fixiert ist eine Drehbewegung von bis zu 12 Grad, bevorzugt von bis zu 24 Grad um die laterale Achse auszuführen, ausgehend von einer horizontalen Lage, welche senkrecht zur Längsachse des Schraubenschafts verläuft.

Die Einzelkomponenten der Pedikelschraube 1 sind vorzugsweise mit einer keramischen Beschichtung versehen.

### Beispiel 3

Eine erfindungsgemäße Vorrichtung zur Stabilisierung der Wirbelsäule besteht aus 5 Pedikelschrauben 1 und einem Verbindungselement 4 mit einer Gesamtlänge von 13 cm.

Diese Vorrichtung kann über insgesamt 5 Wirbel hinweg angebracht werden.

## Patentansprüche

1. Pedikelschraube (1) umfassend einen Schraubenschaft (2) mit einem Kugelkopf (17) sowie einen Schraubenkopf (3) mit zwei gegenüberliegenden Aussparungen, **dadurch gekennzeichnet, dass** der Kugelkopf (17) zwei gegenüberliegende parallele Flächen (18a, 18b) aufweist, die an den entsprechenden parallelen Flächen (18c, 18d) des Schraubenkopfes (3) anliegen und die Längsachse durch die zwei gegenüberliegenden Aussparungen des Schraubenkopfes (3) senkrecht zu den parallelen Flächen (18c, 18d) des Schraubenkopfes (3) verläuft.

2. Pedikelschraube gemäß Anspruch 1, **dadurch gekennzeichnet, dass** in den Schraubenkopf (3) ein Kugelelement (6) mit einer Aussparung zur Aufnahme eines Verbindungselementes (4) beweglich gelagert wird.

3. Pedikelschraube gemäß Anspruch 2, **dadurch gekennzeichnet, dass** das Kugelelement (6) eine Bohrung entlang einer durch die beiden Langlöcher laufenden Achse aufweist.

4. Pedikelschraube gemäß Anspruch 2 oder 3, **dadurch gekennzeichnet, dass** das Kugelelement (6) aus zwei aufeinander gelagerten Oliven (8,9) besteht.

5. Pedikelschraube gemäß eines der vorhergehenden Ansprüche, **dadurch gekennzeichnet, dass** der Schraubenkopf (3) derart auf dem Kugelkopf (17) des Schraubenschafts (2) gelagert ist, dass der Schraubenkopf (3) eindimensionale Kippbewegungen um die axiale Achse ausführen kann.

6. Pedikelschraube gemäß eines der vorhergehenden Ansprüche, **dadurch gekennzeichnet, dass** der Schraubenkopf (3) an der dem Schraubenschaft (2) zugewandten Seite eine konvexe Wölbung (13) besitzt.

7. Pedikelschraube gemäß eines der vorhergehenden Ansprüche, **dadurch gekennzeichnet, dass** der Schraubenkopf (3) an der dem Schraubenschaft (2) gegenüberliegenden Seite eine Öffnung zur Aufnahme eines Fixierungsmittels (5) aufweist.

8. Pedikelschraube gemäß Anspruch 7, **dadurch gekennzeichnet, dass** es sich bei dem Fixierungsmittel (5) um einen Gewindestift handelt.

9. Pedikelschraube gemäß Anspruch 3, **dadurch gekennzeichnet, dass** die dem Fixierungsmittel (5) zugewandte Olive (9) eine Aussparung zur Aufnahme eines Führungsstiftes (11) des Fixierungsmittels (5) aufweist.

10. Vorrichtung zur Stabilisierung der Wirbelsäule umfassend mindestens zwei Pedikelschrauben (1) gemäß eines der Patentansprüche 1 - 7 und mindestens ein Verbindungselement (4).

11. Vorrichtung gemäß Anspruch 10, wobei es sich bei dem mindestens einen Verbindungselement (4) um einen Stützstab, ein Rohr, ein Drahtgeflecht, eine Führungsschiene oder einen gebogenen Führungsstab handelt.

12. Verwendung der Vorrichtung gemäß Anspruch 10 oder 11 zur Stabilisierung der Wirbelsäule.

## Claims

1. Pedicle screw (1), comprising a screw shank (2) with a ball head (17) as well as a screw head (3) having two opposite recesses, **characterized in that** the ball head (17) has two opposite parallel surfaces (18a, 18b) which abut on the corresponding parallel surfaces (18c, 18d) of the screw head (3) and **in that** the longitudinal axis passes through the two opposite recesses of the screw head (3) in perpendicular to the parallel surfaces (18c, 18d) of the screw head (3).

2. Pedicle screw according to claim 1, **characterized in that** a ball element (6) with a recess for receiving a linking member (4) is supported such that it is movable in the screw head (3).

3. Pedicle screw according to claim 2, **characterized in that** the ball element (6) has a bore along an axis which passes through the two elongated holes.

4. Pedicle screw according to claim 2 or 3, **characterized in that** the ball element (6) consists of two olives (8,9) supported on each other.

5. Pedicle screw according to one of the foregoing claims, **characterized in that** the screw head (3) is supported on the ball head (17) of the screw shank (2) such that the screw head (3) can make one-dimensional tilting movements about the axial axis.

6. Pedicle screw according to one of the foregoing claims, **characterized in that** the screw head (3) has a convex curvature (13) on the side faced to the screw shank (2).

7. Pedicle screw according to one of the foregoing claims, **characterized in that** the screw head (3) has an opening for reception of a fixation means (5) on the side which is opposite the screw shank (2).

8. Pedicle screw according to claim 7, **characterized in that** the fixation means (5) is a threaded pin.

9. Pedicle screw according to claim 3, **characterized in that** the olive (9) faced to the fixation means (5) has a recess for reception of a guide pin (11) of the fixation means (5).

10. Device for the stabilization of the spine comprising at least two pedicle screws (1) according to one of the claims 1-7 and at least one linking member (4).

11. Device according to claim 10, wherein the at least one linking member (4) is a support rod, tube, wire netting, guide bar or a bent guide rod.

12. Use of the device according to claim 10 or 11 for the stabilization of the spine.

## Revendications

1. Vis pédiculaire (1) comprenant une tige de vis (2) dotée d'une tête sphérique (17) ainsi qu'une tête de vis (3) dotée de deux ouvertures l'une étant opposée à l'autre, **caractérisé en ce que** la tête sphérique (17) dispose de deux surfaces parallèles l'une opposée à l'autre (18a, 18b) adjacentes aux surfaces parallèles (18c, 18d) correspondantes de la tête de vis (3) et **en ce que** l'axe longitudinal passe, de manière perpendiculaire aux surfaces parallèles (18c, 18d) de la tête de vis (3), à travers des deux ouvertures de la tête de vis (3) l'une étant opposée à l'autre.

2. Vis pédiculaire selon la revendication 1, **caractérisé en ce que** dans la tête de vis (3), un élément sphérique (6) est monté de manière amovible, étant doté d'une ouverture destinée à la réception d'un élément de liaison (4).

3. Vis pédiculaire selon la revendication 2, **caractérisé en ce que** l'élément sphérique (6) est doté d'un alésage tout au long de l'axe passant à travers des deux trous longitudinaux.

4. Vis pédiculaire selon les revendications 2 ou 3, **caractérisé en ce que** l'élément sphérique (6) se compose de deux olives (8, 9) montées l'une au-dessus de l'autre.

5. Vis pédiculaire selon l'une quelconque des revendications précédentes, **caractérisé en ce que** la tête de vis (3) est montée sur la tête sphérique (17) de la tige de vis (2) de manière que la tête de vis (3) peut effectuer des mouvements de bascule unidimensionnels autour de l'axe axial.

6. Vis pédiculaire selon l'une quelconque des revendications précédentes, **caractérisé en ce que** la tête de vis (3) dispose d'une voûte convexe (13) sur le côté tourné vers la tige de vis (2).

7. Vis pédiculaire selon l'une quelconque des revendications précédentes, **caractérisé en ce que** la tête de vis (3) dispose d'une ouverture destinée à la réception d'un moyen de fixation (5) sur le côté opposé à la tige de vis (2).

8. Vis pédiculaire selon la revendication 7, **caractérisé en ce que** le moyen de fixation (5) est une tige filetée.

9. Vis pédiculaire selon la revendication 3, **caractérisé en ce que** l'olive (9) tournée vers le moyen de fixation (5) dispose d'une ouverture destinée à la réception d'une tige de guidage (11) du moyen de fixation (5).

10. Dispositif destiné à la stabilisation de la colonne vertébrale comprenant au moins deux vis pédiculaires (1) selon l'une quelconque des revendications 1 à 7 et au moins un élément de liaison (4).

11. Dispositif selon la revendication 10, le au moins élément de liaison (4) étant une barre de soutien, une tube, un grillage métallique, un rail de guidage ou une barre de guidage courbée.

12. Utilisation du dispositif selon les revendications 10 ou 11 pour stabiliser la colonne vertébrale.
